# EUROPEAN PATENT SPECIFICATION

(11) **EP 2 081 904 B1**
(45) Date of publication and mention of the grant of the patent: **02.03.2011**
(21) Application number: 07818336.5
(22) Date of filing: 21.09.2007
(51) Int. Cl.: C07D 215/22, A61K 31/496, A61P 25/18

(54) **ARIPIPRAZOLE HEMIFUMARATE AND PROCESS FOR ITS PREPARATION**
ARIPIPRAZOLHEMIFUMARAT UND VERFAHREN ZU DESSEN HERSTELLUNG
HEMIFUMARATE D'ARIPIPRAZOLE ET PROCEDE DE PREPARATION ASSOCIE

(30) Priority: 22.09.2006 SI 200600219; 08.06.2007 SI 200700135
(43) Date of publication of application: 29.07.2009
(73) Proprietor: Krka Tovarna Zdravil, D.D., Novo Mesto, 8501 Novo Mesto (SI)
(72) Inventor: MERSLAVIC, Marjo, 8351 Straza pri Novem mestu (SI); GOJAK, Urska, 1000 Ljubljana (SI); SMRKOLJ, Matej, 1420 Trbovlje (SI); BOMBEK, Sergeja, 8000 Novo Mesto (SI); STEFANIC, Marko, 8340 Crnomelj (SI); VAJS, Anamarija, 8000 Novo mesto (SI); KROSELJ, Vesna, 8310 Sentjernej (SI)
(74) Representative: UEXKÜLL & STOLBERG
(86) International application number: PCT/EP2007/008247
(87) International publication number: WO 2008/034628

(56) References cited:
- EP-A- 0 367 141
- US-A1- 2006 223 820

## Description

### FIELD OF THE INVENTION

The present invention relates to aripiprazole hemifumarate, a crystalline and amorphous form thereof, a process for its preparation, a pharmaceutical composition comprising it as well as its use for preparing a pharmaceutical dosage form.

### BACKGROUND OF THE INVENTION

Aripiprazole, having the chemical name 7-[4-[4-(2,3-dichlorophenyl)-1-piperazinyl]butoxy]-3,4-dihydro-2(1H)-quinolinone, and its salts are useful for treating schizophrenia. Therapeutic uses are for example known from EP-A-0 367 141.

EP-A-0 367 141 also describes processes for the preparation of aripiprazole and salts thereof such as the hydrochloride, sulfate, fumarate and maleate salt. Various crystalline forms of aripiprazole and its hydrates are disclosed in WO-A-03/026659, Japanese Unexamined Patent Publication No. 191256/1990, and the 4th Japanese-Korean Symposium on Separation Technology (October 6-8, 1996). New polymorphs and salts are also described in WO-A-2004/083183, WO-A-2004/106322, WO-A-2005/009990, WO-A-2005/016261, WO-A-2005/058835, WO-A-2006/012237, and WO-A-2006/30446. Further, EP-A-1 145 711, EP-A-1 566 174, and WO-A-03/030868 disclose flash-melt dosage forms comprising medicament, superdisintegrant and dispersing agent, wherein CaSiO₃ is the preferred dispersing agent.

However, there still exists a need for a form of aripiprazole which is stable, reproducible and in a suitable form for being further processed into pharmaceutical compositions showing a high content uniformity and improved drug release of aripiprazole.

These objects are solved by aripiprazole hemifumarate according to claims 1 to 9 and 14 to 16. Moreover, the invention is directed to a process for preparing aripiprazole hemifumarate according to claims 10 to 13 and a pharmaceutical composition comprising aripiprazole hemifumarate according to claims 18 to 22 as well as the use of aripiprazole hemifumarate according to claims 23 to 24.

### BRIEF DESCRIPTION OF THE DRAWINGS

Figure 1 is an X-ray powder diffraction pattern of aripiprazole hemifumarate.
Figure 2 is a DSC thermogram of aripiprazole hemifumarate.

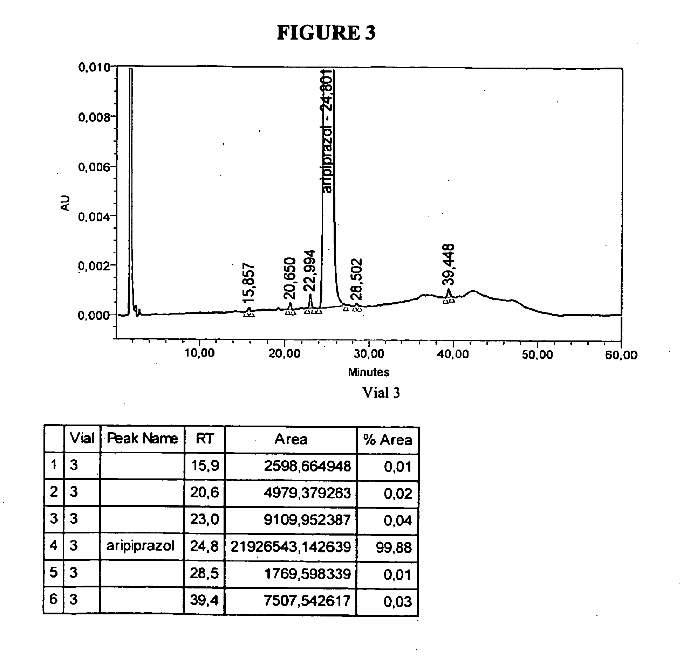

Figure 3 is a HPLC chromatogram of aripiprazole hemifumarate.
Figure 4 is a picture of particles of aripiprazole hemifumarate.
Figure 5 is a particle size distribution diagram of aripiprazole hemifumarate.
Figure 6 is an X-ray powder diffraction pattern of amorphous aripiprazole hemifumarate.

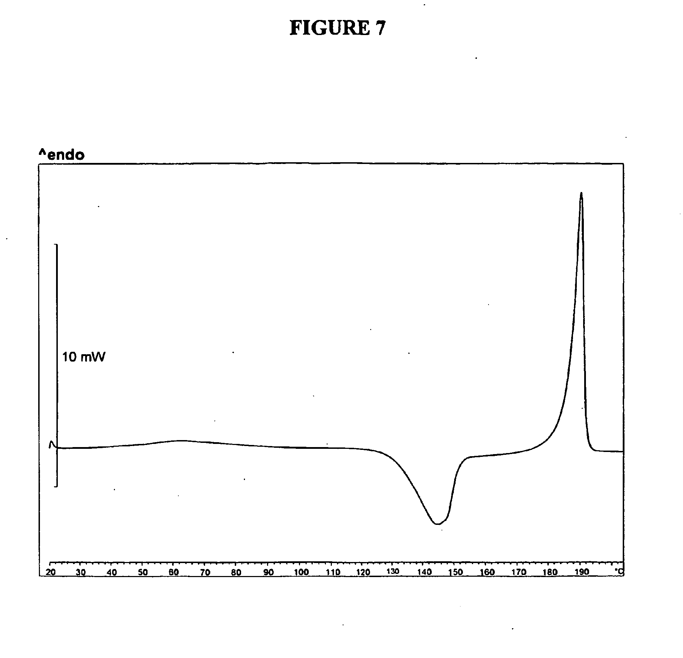

Figure 7 is a DSC thermogram of amorphous aripiprazole hemifumarate.

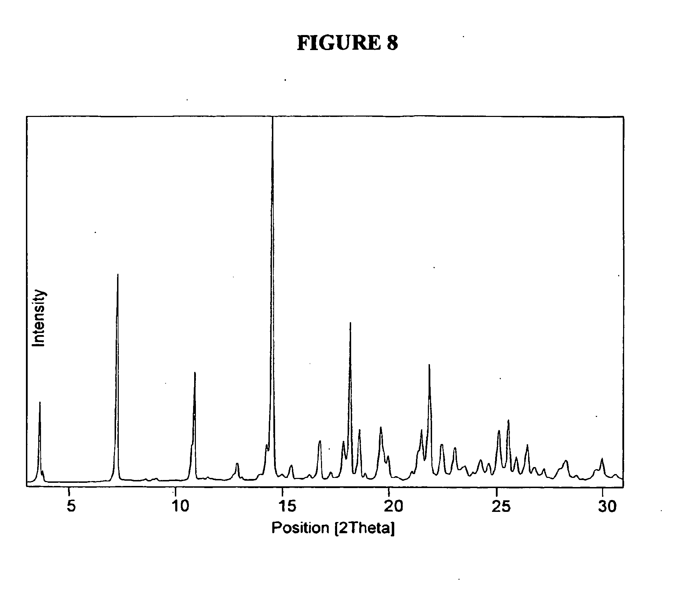

Figure 8 is an X-ray powder diffraction pattern of aripiprazole fumarate (1:1) type I, prepared according to EP-A-0 367 141.

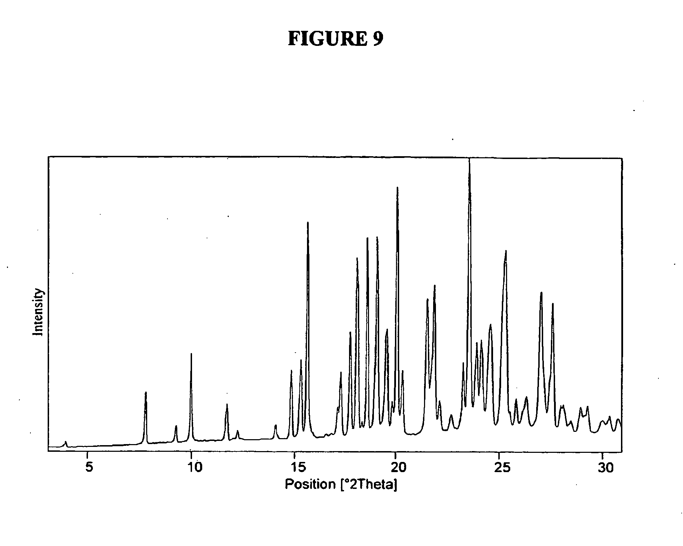

Figure 9 is an X-ray powder diffraction pattern of aripiprazole fumarate (1:1) type II.
Figure 10 is a dissolution profile of aripiprazole from tablets according to example 23 in 900ml 0.1M HCl and in 900ml acetate buffer with pH 4.5.

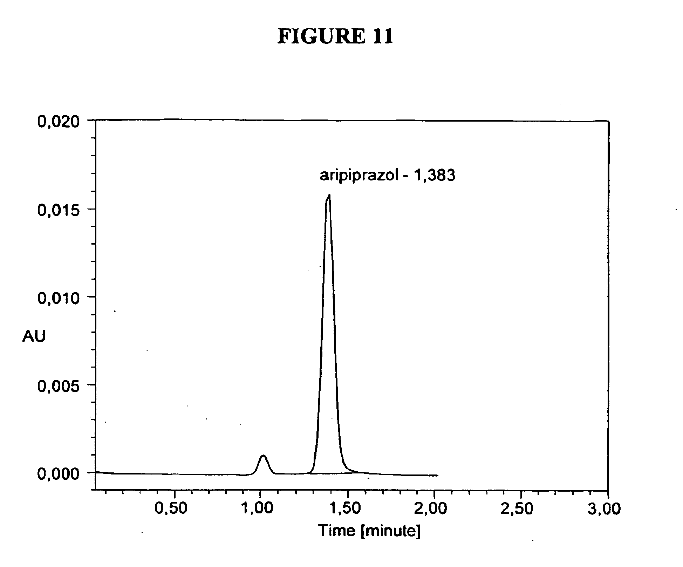

Figure 11 is a HPLC chromatogram of aripiprazole in a solution for dissolution profile determination from tablets according to example 23.
Figure 12 is a picture of particles of aripiprazole type III according to Aoki (Study on Crystal Transformation of Aripiprazole, 4th Japan-Korea Symposium on Separation Technology, p.937 , (1996)).

X-ray powder diffraction patterns were obtained on a Phillips PW3040/60 X'Pert PRO powder diffractometer with CuKα radiation of 1.541874Å.

DSC scans were recorded on a DSC 822e Mettler Toledo scanning calorimeter. Samples of approximately 3 mg were scanned at a heating rate of 10°C/min under nitrogen atmosphere.

HPLC chromatogram of aripiprazole hemifumarate salt was obtained on a Waters Alliance 2690 chromatograph with PDA 996 detector. Chromatographic separation was acheived using Inertsil ODS-3, 250x4.6mm 5µm analytical column. Chromatographic conditions used were: sample concentration of 1mg/ml, detection at 25.0nm, flow of 1.5ml/min, volume of injection 20µL. A gradient elution using mobile phase A (0.1M KH₂PO₄ : CH₃CN = 81:19 (V/V)) and mobile phase B (0.1M KH₂PO₄ : CH₃CN = 50:50 (V/V)) was employed with time table (0min 10%B, 40min 90%B, 45min 90%B).

HPLC evaluation of dissolution samples was performed with Gemini C18 110Å, 100 x 4.6 mm i.d., 5 µM analytical column. Chromatographic conditions used were: detection at 250 nm, mobile phase 0.005M KH₂PO₄:CH₃CN = 40:60 (V/V), flow of 1.0 ml/min, injection volume of 5 µl and column temperature of 30°C.

The size distribution of aripiprazole hemifumarate particles was determined by laser diffraction using a Malvern Mastersizer 2000 laser diffraction instrument. The samples for analysis were prepared by dispersing a weighed amount of aripiprazole hemifumarate particles in isopar. The term "average particle size" therefore represents the mean particle diameter.

### DETAILED DESCRIPTION OF THE INVENTION

A new salt of aripiprazole, namely aripiprazole hemifumarate, has been found which differs from aripiprazole fumarate disclosed in EP-A-0 367 141 in that the ratio between aripiprazole and fumarate is 2:1.

Thus, the present invention relates to aripiprazole hemifumarate which is characterized in that the molar ratio between aripiprazole and fumarate is 2:1. The new salt is stable, reproducible and useful for the preparation of pharmaceutical formulations.

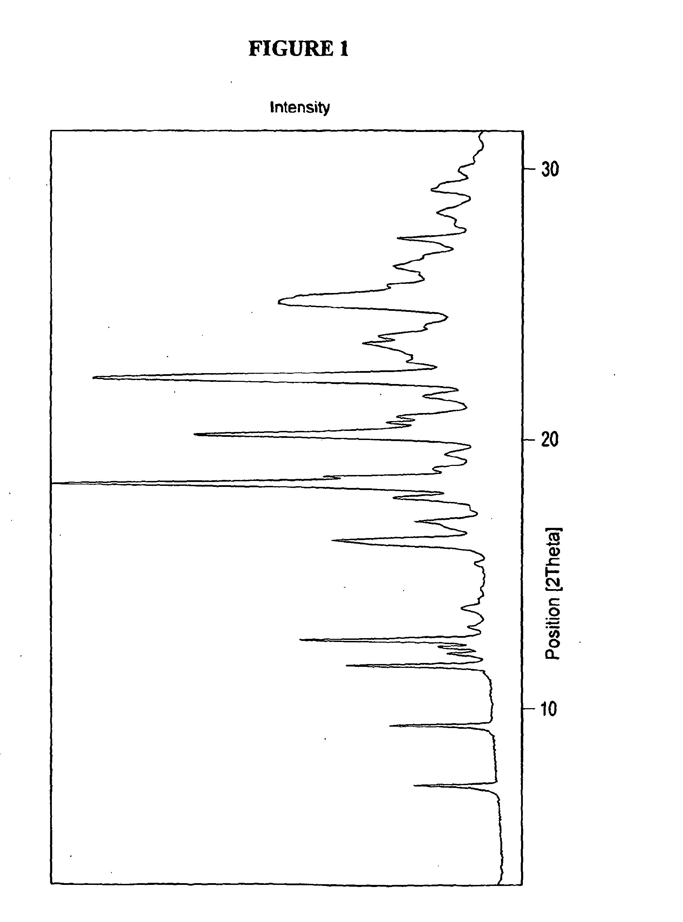

In a preferred embodiment of the present invention the aripiprazole hemifumarate is crystalline and can be characterized by an X-ray powder diffraction pattern having peaks at about 12.1, 15.8, 17.9, 19.7, 21.8 ± 0.2 degrees two-theta. Aripiprazole hemifumarate can be further characterized by X-ray powder diffraction peaks at about 6.7, 8.9, 11.1, 12.1, 15.8, 17.9, 19.7, 21.8, 24.6 ± 0.2 degrees two-theta. It may either be of up to 100% polymorphic purity or include other polymorphic, or an amorphous form. The X-ray powder diffraction pattern of crystalline aripiprazole hemifumarate according to the invention is shown in Figure 1.

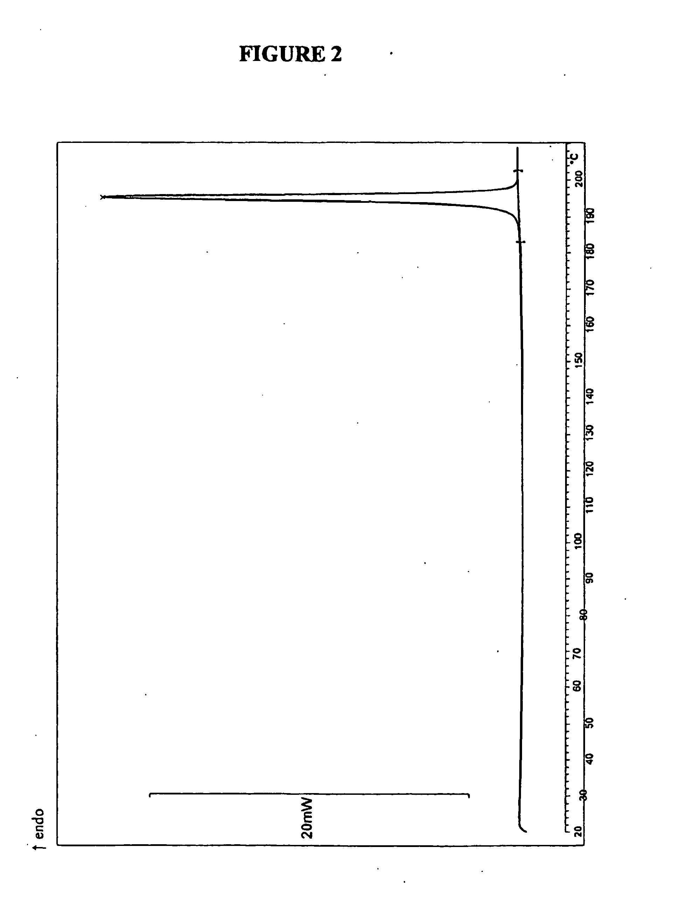

The aripiprazole hemifumarate according to the present invention can also be characterized by having a DSC endothermic peak at about 187-192°C. An example of a DSC thermogram of aripiprazole hemifumarate according to the invention is shown in Figure 2.

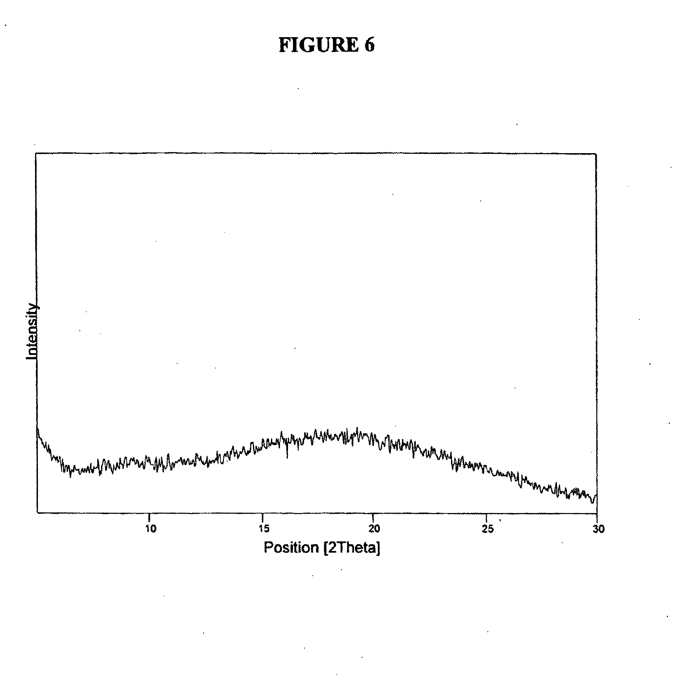

According to another embodiment of the present invention, the aripiprazole hemifumarate is amorphous. Preferably, amorphous aripiprazole hemifumarate is characterized by an X-ray powder diffractiogram as depicted in Figure 6.

The present invention is also directed to a process for preparing aripiprazole hemifumarate. The process for preparing aripiprazole hemifumarate is characterized in that it comprises:
a) dissolving aripiprazole in a solvent at a temperature of between 15 °C and the reflux temperature of the solvent used,
b) adding fumaric acid in a molar ratio of 0.5 - 0.7 with regard to aripiprazole and heating the mixture to a temperature of between 15 °C and the reflux temperature of the solvent used,
c) optionally cooling the obtained solution and
d) optionally filtering the precipitated solid.

Preferably, this process results in crystalline aripiprazole hemifumarate. The obtained crystalline product can be converted into amorphous aripiprazole by techniques known in the art such as heating crystalline aripiprazole above its melting point and rapidly cooling.

The fumaric acid added in step b) can be in the form of a solution or in the solid form.

In another aspect of the present process for the preparation of aripiprazole hemifumarate the order of adding aripiprazole and fumaric acid may also be reversed and aripiprazole is added to a solution of fumaric acid. Aripiprazole may be added in the form of a solution or in the solid form.

According to a preferred embodiment of the process of the invention, no further solvent and in particular no organic solvent such as an anti-solvent is added after step b), i.e. after fumaric acid is added to a solution of aripiprazole or after aripiprazole is added to a solution of fumaric acid.

Aripiprazole base can be prepared according to methods disclosed in EP-A-0 367 141, and can also be further recrystallized and/or purified up to a level of 99.9 %. The aripiprazole used in the preparation of hemifumarate salt can be either pure or crude. If the aripiprazole hemifumarate prepared by the present invention is not satisfactorily pure, it can be recrystallized up to a purity level of 99.9 %.

Suitable solvents for crystallizing aripiprazole hemifumarate, and in particular suitable solvents that can be used in step a) and/or b) of the process according to the invention for preparing aripiprazole hemifumarate, include organic solvents such as hydrocarbons (e.g. pentane, hexane, heptane), halogenated hydrocarbons (e.g. methylene chloride) , nitriles (e.g. acetonitrile), ketones, ethers (e.g. THF ), esters and alcohols (e.g. methanol, ethanol, isopropanol, propanol, butanol).

According to one embodiment of the process of the invention, the hemifumarate salt can be isolated in a solvated form. The solvent can then be removed by drying. As used.herein, the term "drying" generally refers to a removal of solvent until aripiprazole hemifumarate contains less than about 6000 ppm of residual solvents. Drying may be achieved e.g. via application of heat, preferably carried out under ambient or reduced pressure or via contacting aripiprazole hemifumarate with humid or dry air in a fluidized bed drier, wherein the atmosphere in the fluidized bed drier has at least 15% humidity. The term "reduced pressure" refers to a pressure below one atmosphere, more preferably below about 100 mmHg.

The particle size of aripiprazole hemifumarate prepared according to the present invention can be in the range of 0.1-250 microns. For example, the size of the prepared aripiprazole hemifumarate particles depends on the rate of cooling of the solution, if applicable.

According to one embodiment of the process of the present invention, and in particular if smaller particles are needed, the obtained aripiprazole hemifumarate can be sieved, milled or micronized optionally together with other excipients. In this process the mechanical force on the particle surface leads to particle size reduction, however it may also release structure changes of the material. For example, air jet mill, ball mill or hammer mill are commonly used as milling equiment.

The basic principle of treatment in an air jet mill is collision and attrition between particles suspended within the high velocity air stream, which introduces the power to the milling chamber. In a ball mill, the particles are fractured by impact of grinding media (balls, cubes, cylinders, etc.) that can occupy up to half of the mill chamber volume. Due to rotation of the chamber the grinding media is falling from an elevated position. Friction is also present among all elements, contributing significantly to the attrition and consequently to amorphous nature of the material being milled. One of the most widely used mills in the pharmaceutical industry is the hammer mill. In such equipment, particles are exposed to the impact of rapidly rotating hammers. During milling material additionally hit the perforated screen that is placed over the chamber outlet.

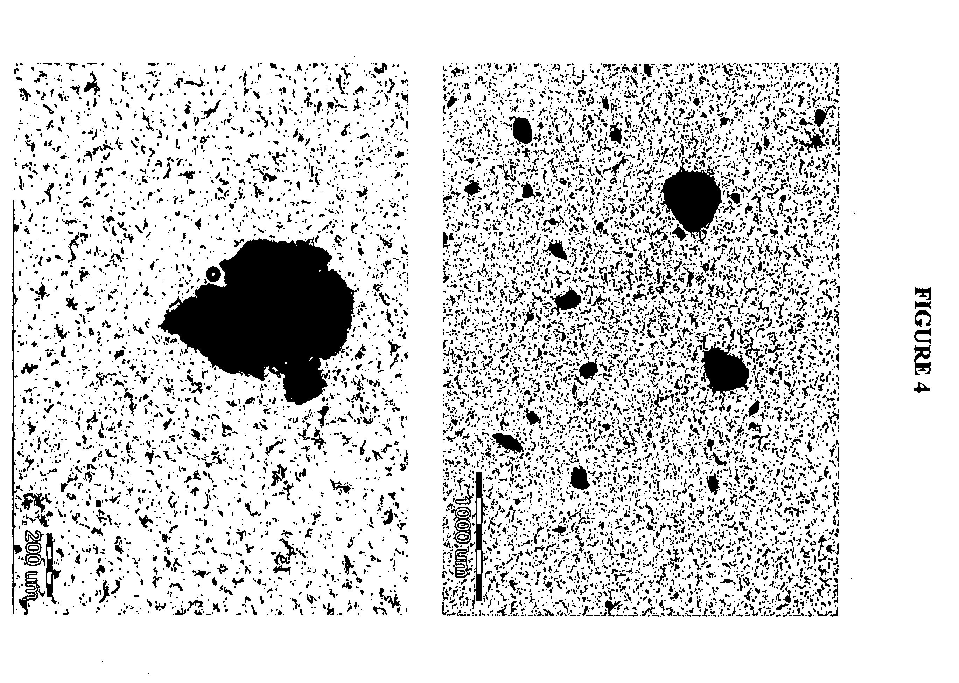

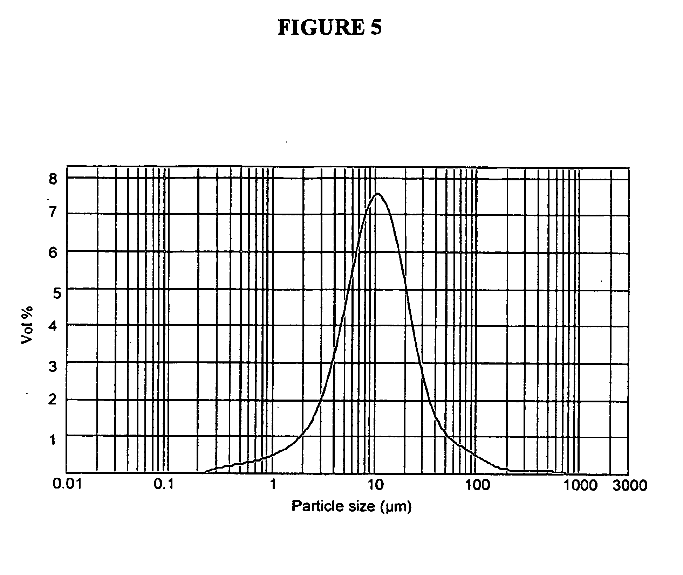

Very fine particles of the active ingredient can spontaneously agglomerate due to Van der Waals and electrostatic forces. Figure 4 depicts small particles of aripiprazole hemifumarate with irregular shapes and fused into agglomerates. According to the process of the present invention particles of the active ingredient are obtained without agglomerates, which are perfectly suitable for formulation. An example of a particle size distribution of aripiprazole hemifumarate according to the invention is shown on Figure 5.

Agglomerates of the active ingredient have great influence on the physico-chemical properties of tablets. Because of agglomeration, the content of the active ingredient can be nonuniformly distributed and drug release from tablets can be slower. Thus, it is desired to prevent the agglomeration or break the agglomerates into small particles using suitable procedures. According to one embodiment of the process of the present invention this is achieved by sieving the obtained aripiprazole hemifumarate. In particular, the active ingredient aripiprazole hemifumarate is sieved with a part of a diluent such as maize starch or lactose through a sieve having a nominal sieve opening of about 0.2 to about 1.0 mm, preferably 0.4 to 0.8. mm and most preferably about 0.6 mm before the granulation process. In addition, agglomerates of aripiprazole hemifumarate can also be breaked by any other methods, such as ultrasound or milling.

In a preferred embodiment, the aripiprazole hemifumarate according to the present invention is substantially free of agglomerates. In particular, the average particle size of aripiprazole hemifumarate according to the present invention is below 70 micrometers, preferably below 50 micrometers, most preferably below 30 micrometers. The most preferred average particle size of aripiprazole hemifumarate is below 20 micrometers.

The present invention also relates to pharmaceutical compositions containing aripiprazole hemifumarate according to the invention as active ingredient in admixture with one or more carriers or auxiliary substance(s) conventionally applied in the pharmaceutical industry.

The pharmaceutical compositions according to the invention usually contain 1 - 90 % by weight, preferably 2 - 50 % by weight, most preferably 3 - 30 % by weight of the active ingredient. Further, the pharmaceutical compositions can be characterized in that they contain an amount of aripiprazole hemifumarate which corresponds to 1-50 mg of aripiprazole per dosage unit. Most preferably, the pharmaceutical compositions according to the present invention contain an amount of aripiprazole hemifumarate which corresponds to 2 mg, 5 mg, 10 mg, 15 mg, 20 mg or 30 mg of aripiprazole per dosage unit.

The pharmaceutical compositions of the present invention may be suitable for oral (for example powders, tablets, coated tablets, capsules, orally disintegrating tablets, chewable tablets, solutions, suspensions or emulsions), parenteral (for example injection solutions for intravenous, intramuscular, subcutaneous or intraperitoneal use), rectal (suppositories), transdermal (patches) or local (ointments or patches) administration or for the application in form of implants. The solid, semisolid or liquid pharmaceutical compositions according to the invention may be produced by methods conventionally applied in the pharmaceutical industry.

A solid pharmaceutical composition further comprises at least one auxiliary agent selected from the group of fillers or carriers, binding agents, diluents, disintegrants, lubricants, glidants, surface active agents, sweetening agents, flavours, stabilizers etc.

Diluents can be selected (but are not limited to) from the group of lactose in different forms (anhydrous, monohydrate, spray dried lactose etc.), microcrystalline cellulose (such as commercially available Avicel PH 101, Avicel PH 102 or Avicel PH 112), powdered cellulose, silicified microcrystalline cellulose, calcium phosphate, calcium hydrogen phosphate, calcium carbonate, sucrose, glucose, fructose, dextrates, maltodextrins, other sugars such as mannitol, lactitol, xylitol, sorbitol, calcium lactate or combined diluents (e.g. F-MELT). Starches such as pregelatinized starch can also be used as a diluent.

The composition according to the invention can also comprise binders, such as povidone, microcrystalline cellulose, hydroxyethylcellulose, hydroxypropylcellulose, low-substituted hydroxypropylcellulose (comprising 5 to 16% by weight of hydroxypropyl groups), hydroxypropylmethylcellulose or other cellulose ethers, starch, pregelatinized starch, gelatine, polymethacrylate or mixture of binders.

Further, disintegrants and/or superdisintegrants may also be present such as starches (e.g. maize starch, potato starch), modified starches (sodium starch glycolate), modified cellulose (croscarmellose, i.e. cross-linked carboxymethlycellulose sodium), cross-linked polyvinylpyrrolidone (crospovidone), microcrystalline cellulose, carboxymethylcellulose sodium, Amberlite^{®}, alginic acid, sodium alginate, guar gum, gellan gum, Xanthan SM^{®}, calcium silicate, magnesium silicate, magnesium trisilicate, magnesium aluminometasilicate (e.g. Neusilin^{®}) and other magnesium salts. It is preferred that excipients include at least one disintegrant or superdisintegrant selected from croscarmellose, crospovidone and microcrystalline cellulose.

Further, lubricants may also be present as excipients, such as stearic acid, magnesium stearate, calcium stearate, sodium laurylsulphate, hydrogenated vegetable oil, hydrogenated castor oil, sodium stearyl fumarate, talc, macrogols. It is preferred that the excipients include at least one lubricant selected from magnesium stearate, sodium stearyl fumarate or hydrogenated vegetable oil.

Glidants can also be added to the composition according to the invention. They can be selected from the group consisting of talc, silicon dioxide of different grades (such as colloidal or precipitated silica) etc.

Excipents may have multiple functions, i.e. one excipient may be diluent and additionally binder, binder and disintegrant etc.

Optionally, surfactants can be included in a solid pharmaceutical formulation. Surfactants can be selected from the group of non-ionic or ionic surfactants or mixtures thereof. Suitable non-ionic surfactants are selected from the group of alkylglucosides, alkylmaltosides, alkylthioglukosides, lauryl macrogolglycerides, polyoxyethylene alkylphenols, polyoxyethylene alkylethers, polyethylene glycol fatty acid esters, polyethylene glycol glycerol fatty acid esters, polyoxyethylene sorbitan fatty acid esters, polyoxyethylene-polyoxypropylene block copolymers, polyglyceryl fatty acid esters, polyoxyethylene glycerides, polyoxyethylene vegetable oils, polyoxyethylene hydrogenated vegetable oils, sterols, and mixtures thereof. Preferred non-ionic surfactants are polyoxyethylene sorbitan fatty acid esters which are sold under the trade names Polysorbate or Tween.

Suitable ionic surfactants are selected from group of fatty acid salts, bile salts, phospholipides, phosphoric acid esters, carboxylates, sulphates, sulphonates and mixture thereof. A preferred ionic surfactant is sodium laurylsulphate.

Further, the composition can comprise sweetening agents. Examples of sweeteners are aspartame, acesulfam K, saccharin sodium, dipotassium glycyrrhizinate, stevia and thaumatin.

Flavouring agents such as natural or synthetic materials, e.g. vanilla flavour, can also be contained in the pharmaceutical composition according to the invetion.

Generally, any suitable process for the preparation of pharmaceutical compositions may be applied for preparing the pharmaceutical composition according to the invention, e.g. direct compression, wet (water or alcohol) or dry granulation etc. A preferred process for the preparation of the pharmaceutical compositions containing aripiprazole hemifumarate as active ingredient comprises admixing said active ingredient with at least one carrier(s) or auxiliary substance(s) conventionally applied in the pharmaceutical industry and bringing the mixture to a galenic form.

The pharmaceutical composition of aripiprazole hemifumarate can be processed and packed under a modified atmosphere. An atmosphere having a modified oxygen content or reduced oxygen partial pressure can be obtained by the use of a reduced pressure atmosphere, e.g. by creating a partial vacuum by means of a suitable pump or by partial freezing or liquefying the atmosphere, or by the use of an inert gas, such as nitrogen or argon, or by the use of oxygen absorbents, even though the high efficacy of such absorbents leading to very low oxygen levels is normally not necessary.

The pharmaceutical composition according to the invention can be stored in a packaging, with a blister packaging or a bottle being preferred. In that case a packaged composition is formed. The packaging can be provided by means for trapping and disposal of free oxygen. The composition can be enclosed in a substantially gas exchange non-permeable material as packaging which has an atmosphere having the required reduced oxygen content. The substantially gas exchange non-permeable packaging is preferably selected from the group consisting of an Al/Al blister package, an Al-polychloro-3-fluoroethylene homopolymer/PVC laminate blister or a bottle.

The pharmaceutical composition according to the invention has such a dissolution profile, that at least 80% of aripiprazole hemifumarate is dissolved in 0.1M HCl in 30 minutes or at least 80% of aripiprazole hemifumarate is dissolved in pH 4.5 acetate buffer in 30 minutes.

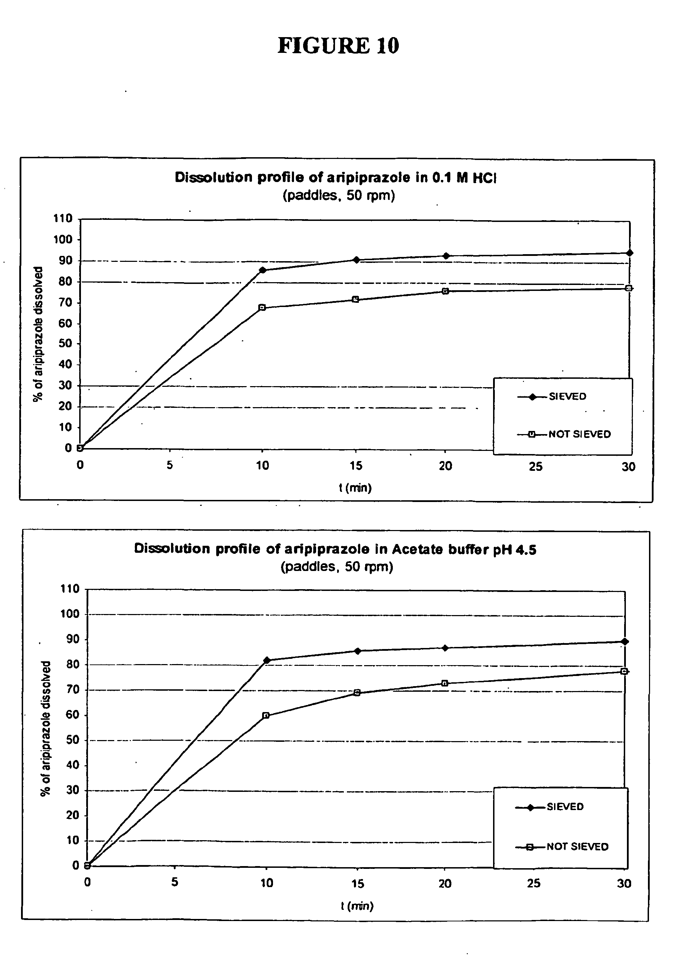

Studies on the content uniformity and dissolution of aripiprazole from tablets according to Example 23 are shown below. Figure 10 shows dissolution profiles of aripiprazole in 900 ml of 0.1 M HCl and in 900 ml of acetate buffer having a pH of 4.5, respectively. Both profiles show that sieving positively effects dissolution, since dissolution of sieved aripiprazole hemifumarate is 17-19% faster in 0.1M HCl and 12-22% in acetate buffer.

The invention is in the following explained in more detail with reference to examples and figures.

### EXAMPLES

### I. SYNTHESIS

### Example 1: Preparation of aripiprazole hemifumarate

4.8 g of 7-(4-(4-(2,3-dichlorophenyl)-1-piperazinyl)-3,4-dihydrocarbostyril obtained as described in EP-B-0 367 141, example 1, were dissolved in 72 ml of ethanol. To this solution 0.6 g of fumaric acid were added. The suspension was maintained at reflux for 15 minutes and then allowed to cool over 3 hours to room temperature. The precipitate was collected by filtration and dried at 50°C under 40 mm Hg to give aripiprazole hemifumarate in 94.8 % yield.
Melting range : 187 -192°C.
1H NMR (DMSO) δ 1.60 - 1.72 ( 4H, m) , 2,38- 2.47 (4H, m) , 2.59 ( 4H, bm), 2.99 ( 4H, bm),
3.92 ( 2H, t), 6.43 - 6.44 ( 1H, d), 6.45 - 6.50 ( 1H, dd), 6.60 (1H, s), 7.02 - 7.05 (1H, d), 7.12 - 7.15 (1H, m), 7.29 - 7.31 (2H, m) and 9.98 (1H, s).

### Example 2: Preparation of aripiprazole hemifumarate from tetrahydrofurane

1.2 g of 7-(4-(4-(2,3-dichlorophenyl)-1-piperazinyl)-3,4-dihydrocarbostyril were dissolved in 25ml of tetrahydrofurane. To this solution 0.127 g of fumaric acid were added. The suspension was maintained at 20 - 25°C for 12 hours. The precipitate was collected by filtration and dried at 50°C under 40 mm Hg to give aripiprazole hemifumarate in 62.7 % yield.

### Example 3: Preparation of aripiprazole hemifumarate from acetonitrile

1.2 g of 7-(4-(4-(2,3-dichlorophenyl)-1-piperazinyl)-3,4-dihydrocarbostyril were suspended in 25 ml of acetonitrile. To this suspension 0.127 g of fumaric acid were added. The suspension was maintained at 20 - 25°C for 12 hours. The precipitate was collected by filtration and dried at 50°C under 40 mm Hg to give aripiprazole hemifumarate in 89 % yield.

### Example 4: Preparation of aripiprazole hemifumarate from methylene chloride

1.2 g of 7-(4-(4-(2,3-dichlorophenyl)-1-piperazinyl)-3,4-dihydrocarbostyril were dissolved in 25 ml of methylene chloride. To this solution 0.127 g of fumaric acid were added. The suspension was maintained at 20 - 25°C for 12 hours. The precipitate was collected by filtration and dried at 50°C under 40 mm Hg to give aripiprazole hemifumarate in 76.8 % yield.

### Example 5: Synthesis of crude aripiprazole

A suspension of dichloromethane (50 ml) and 1-(2,3-dichlorophenyl)piperazine hydrochloride (4.65 g) was stirred for 10 minutes, then a solution of NaOH was added (0,71 g in 20 ml of water; pH 7 to 8). After stirring for 30 minutes the layers were separated and aqueous layer was extracted once with 10 ml of dichloromethane. The organic layers were evaporated in vacuo to give 4 g of 1-(2,3-dichlorophenyl)piperazine as oil. To a solution of 1-(2,3-dichlorophenyl)piperazine (4 g) in acetonitrile (20 ml) and water (30 ml) 4.8 g of 7-(4-(4-(2,3-dichlorophenyl)-1-piperazinyl)-3,4-dihydrocarbostyril, 0.2 g of sodium iodide and 2.6 ml of triethylamine were added during strong stirring. The whole mixture was further refluxed for 3 hours and slurried for 1 hour at ambient temperature. The product was filtered and washed with water (10ml).

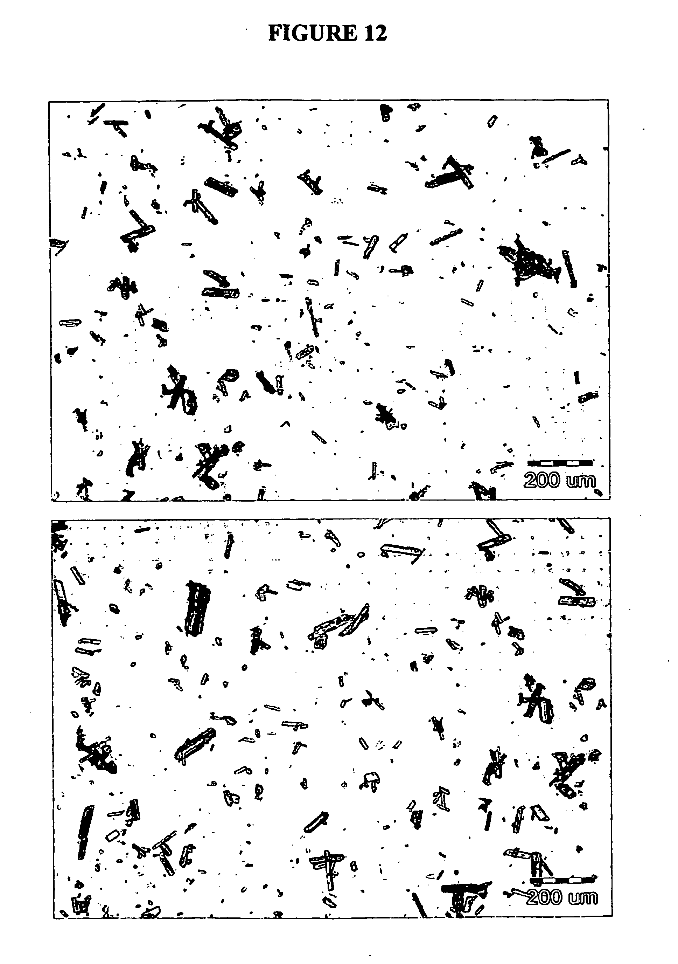

The obtained crude aripiprazole was of type III acording to Aoki (Study on Crystal Transformation of Aripiprazole, The Fourth Japan-Korea Symposium on Separation Technology, p.937 , (1996)). Images of particles of aripiprazole type III are shown in Figure 12.

### Example 6: Preparation of aripiprazole hemifumarate from ethanol

Crude aripiprazole (2g) was dissolved in ethanol (20 ml) at reflux . A solution of fumaric acid (0.26 g, 50°C) in ethanol-(5 ml) was added to this solution. The suspension was aged at reflux for 15 minutes, allowed to cool to room temperature and stirred at room temperature for 2 -3 hours. The precipitated crystals were collected by filtration, washed with ethanol (2 ml) and dried at 60°C. Yield: 2.15 g (95 %), average particle size: 29 µm, specific surface area: 2,4 m²/g.

### Example 7: Preparation of aripiprazole hemifumarate from ethanol-water

Crude aripiprazole (1 g) was suspended in ethanol (10 ml) and water (30 ml). The suspension was heated to reflux to form a solution and fumaric acid (0.15 g) was added. The suspension was aged at reflux for 10 minutes, allowed to cool to room temperature and stirred at room temperature for 2 to 3 hours. The precipitated crystals were collected by filtration, washed with water/ethanol (5 ml) and dried at room temperature. Yield: 1.08 g (96 %).

### Example 8 (Reference Example): Preparation of aripiprazole fumarate (1:1) type II

Crude aripiprazole (2 g) was suspended in acetonitrile (30 ml) and water (0.4 ml). The suspension was heated to reflux to form a solution and fumaric acid (0.58 g) was added. The suspension was aged at reflux for 10 minutes, allowed to cool to room temperature and stirred at room temperature for 12 hours. The precipitated crystals were collected by filtration, washed with acetonitrile (5 ml) and dried at room temperature. Yield: 2.47 g (98 %).

### II. PHARMACEUTICAL FORMULATIONS

### ORALLY DISINTEGRATING TABLETS

### A.) Dry granulation

### Example 9: 5 mg orally disintegrating tablets

| | Amount / mg | Amount / % (by weight) |
|---|---|---|
| Aripiprazole hemifumarate | 5.65* | 7.53 |
| Xylitol | 39.45 | 52.60 |
| Microcrystalline cellulose | 10.00 | 13.33 |
| Calcium silicate | 11.25 | 15.00 |
| Crospovidone | 5.00 | 6.67 |
| Amorphous silica | 1.50 | 2.00 |
| Aspartame | 1.00 | 1.33 |
| Flavour Vanilla | 0.40 | 0.54 |
| Magnesium stearate | 0.75 | 1.00 |
| Total | 75.00 | 100 |

| | | |
|---|---|---|
| *corresponding to 5 mg of aripiprazole | | |

Aripiprazole hemifumarate was mixed in a high shear mixer with all other excipients (except a part of magnesium stearate), 0.25 % of magnesium stearate was added, the mixture was compacted with a roller compactor, the obtained compacts were milled and sieved, blended with the remaining magnesium stearate and compressed into tablets. For the differentiation between different strengths, part of microcrystalline cellulose can be replaced by different colouring agents, such as iron oxides.

### B.) Wet granulation

### Example 10: 10 mg orally disintegrating tablets

| | Amount / mg | Amount / % (by weight) |
|---|---|---|
| Aripiprazole hemifumarate | 11.30* | 7.53 |
| Mannitol | 81.90 | 54. 60 |
| Microcrystalline cellulose | 12.00 | 8.00 |
| Low substituted HPC | 9.00 | 6.00 |
| Aspartame | 1.00 | 0.67 |
| Flavour Vanilla | 0.80 | 0.53 |
| Crospovidone | 12.00 | 8.00 |
| Calcium silicate | 20.00 | 13.34 |
| Magnesium stearate | 2.00 | 1.33 |
| Total | 150 | 100 |
| Purified water | q.s. | evaporates during the process |

| | | |
|---|---|---|
| * corresponds to 10 mg of aripiprazole "q.s." means "as needed" | | |

Mannitol, microcrystalline cellulose, low substituted HPC, aspartame and crospovidone were mixed in a suitable mixer and granulated with purified water. The obtained granulate was dried, sieved and mixed with aripiprazole hemifumarate, flavour vanilla and calcium silicate. In the end, magnesium stearate was admixed and the obtained mixture was compressed to tablets at < 60 N. The disintegration times were in the range of 10 to 20 s (purified water at 37°C, Ph. Eur.).

### Example 11: 10 mg orally disintegrating tablets

| | Amount / mg | Amount / % (by weight) |
|---|---|---|
| Aripiprazole hemifumarate | 11.30* | 7.53 |
| Mannitol | 17.70 | 11.80 |
| Microcrystalline cellulose (Avicel PH 101) | 50.00 | 33.33 |
| Pregelatinized starch | 7.50 | 5.00 |
| Aspartame | 2.00 | 1.33 |
| Flavour Vanilla | 1.00 | 0.67 |
| Crospovidone | 15.00 | 10.00 |
| Colloidal silicon dioxide | 2.00 | 1.33 |
| Microcrystalline cellulose (Avicel PH 112) | 35.00 | 23.34 |
| Microcrystalline cellulose and guar gum (Avicel CE 15) | 7.00 | 4.67 |
| Sodium lauryl sulphate | 0.50 | 0.33 |
| Magnesium stearate | 1.00 | 0.67 |
| Total | 150 | 100.00 |
| Purified water | q.s. | evaporates during the process |

| | | |
|---|---|---|
| * corresponds to 10 mg of aripiprazole "q.s." means "as needed" | | |

Aripiprazole hemifumarate, mannitol, microcrystalline cellulose (Avicel PH 101), pregelatinized starch and half of the crospovidone were mixed in a suitable granulator and granulated with the solution of sodium lauryl sulphate in water. The granules were dried and sieved, all other ingredients were added and the lubricated blend was compressed to tablets at < 60 N. The disintegration times were in the range of 10 to 20 s (purified water at 37°C, Ph. Eur.).

### Example 12: 10 mg orally disintegrating tablets

| | Amount / mg | Amount / % (by weight) |
|---|---|---|
| Aripiprazole hemifumarate | 11.30* | 7.53 |
| Xylitol | 55.91 | 37,27 |
| Microcrystalline Cellulose | 30.48 | 20.32 |
| Low substituted HPC | 9.00 | 6.00 |
| Crospovidone | 10.00 | 6,67 |
| Mannitol | 30.00 | 20.00 |
| Flavour Vanilla | 0.81 | 0.54 |
| Aspartame | 1.00 | 0.67 |
| Mg-stearate | 1.50 | 1.00 |
| Total | 150.00 | 100 |
| Purified water | q.s. | evaporates during the process |

| | | |
|---|---|---|
| *corresponding to 10 mg of aripiprazole "q.s." means "as needed" | | |

Xylitol, mannitol, microcrystalline cellulose, low substituted HPC, aspartame and crospovidone were blended in a suitable mixer and granulated with purified water. The obtained granulate was dried, sieved and mixed with aripiprazole hemifumarate and flavour vanilla. In the end, magnesium stearate was admixed and the obtained mixture was compressed into round tablets.

### Example 13: 10 mg orally disintegrating tablets

| | Amount / mg | Amount / % (by weight) |
|---|---|---|
| Aripiprazole hemifumarate | 11.30* | 7.53 |
| Sucrose | 65.27 | 43.51 |
| Povidone | 4.35 | 2.90 |
| Silica, colloidal anhydrous | 1.09 | 0.73 |
| Sodium starch glycolate | 10.00 | 6.67 |
| Corn starch | 53.73 | 35.82 |
| Aspartame | 0.69 | 0.46 |
| Flavour Vanilla | 2.07 | 1.38 |
| Magnesium stearate | 1.50 | 1.00 |
| Total | 150.00 | 100 |
| Purified water | q.s. | evaporates during the process |

| | | |
|---|---|---|
| *corresponding to 10 mg of aripiprazole "q.s." means "as needed" | | |

Aripiprazole hemifumarate, sucrose and corn starch were mixed in a granulator. Granulating liquid was prepared by dissolving povidone in purified water and sprayed onto a powder mixture. The obtained granulate was dried and sieved. In the end, flavour vanilla, aspartame, sodium starch glycolate, silica and magnesium stearate were admixed and the obtained mixture was compressed into tablets.

### Example 14: 10 mg orally disintegrating tablets

| | Amount / mg | Amount / % (by weight) |
|---|---|---|
| Aripiprazole hemifumarate | 11.30* | 7.53 |
| Xylitol | 80.47 | 53.65 |
| Magnesium aluminosilicate | 22.50 | 15.00 |
| Microcrystalline cellulose | 22.50 | 15.00 |
| Aspartame | 3.00 | 2.00 |
| Flavour Vanilla | 0.23 | 0.15 |
| Crospovidone | 9.00 | 6.00 |
| Magnesium stearate | 1.00 | 0.67 |
| Total | 150.00 | 100.00 |
| Purified water | q.s. | evaporates during the process |

| | | |
|---|---|---|
| corresponds to 10 mg of aripiprazole "q.s." means "as needed" | | |

Aripiprazole hemifumarate, xylitol, magnesium aluminosilicate, a part of microcrystalline cellulose, aspartame, flavour and crospovidone were blended in the suitable mixer and granulated with purified water. The obtained granulate was dried, sieved and homogeneously mixed with the rest quantity of microcrystalline cellulose and magnesium stearate. The obtained compression mixture was compressed into tablets.

### C.) Direct Compression

### Example 15: 10 mg orally disintegrating tablets

| | Amount / mg | Amount / % (by weight) |
|---|---|---|
| Aripiprazole hemifumarate | 11.30* | 7.53 |
| Maltodextrin (Maltrin QD) | 75.90 | 50.60 |
| Microcrystalline cellulose (Avicel PH 105) | 10.00 | 6.67 |
| Microcrystalline cellulose (Avicel PH 102) | 10.00 | 6.67 |
| Aspartame | 1.00 | 0.67 |
| Flavour Vanilla | 0.80 | 0.53 |
| Crospovidone | 10.00 | 6.67 |
| Calcium silicate | 30.00 | 20.00 |
| Magnesium stearate | 1.00 | 0.66 |
| Total | 150 | 100 |

| | | |
|---|---|---|
| * corresponds to 10 mg of aripiprazole | | |

All ingredients, except magnesium stearate, were mixed in a suitable mixer. Magnesium stearate was added in the end and the lubricated compression mixture was pressed into tablets using round punches. The disintegration time was approx. 20 s (purified water at 37°C, Ph. Eur.) at a hardness of about 50 N.

### Example 16: 10 mg orally disintegrating tablets

| | Amount / mg | Amount / % (by weight) |
|---|---|---|
| Aripiprazole hemifumarate | 11.30* | 7.53 |
| Pharmburst^{™} | 133.00 | 88.67 |
| Acesulfame K | 0.22 | 0.15 |
| Aspartame | 0.23 | 0.15 |
| Flavour Vanilla | 2.25 | 1.50 |
| Magnesium stearate | 1.50 | 1.00 |
| Talc | 1.50 | 1.00 |
| Total | 150.00 | 100 |

| | | |
|---|---|---|
| *corresponding to 10 mg of aripiprazole | | |

All ingredients, except magnesium stearate, were homogeneously blended in a suitable mixer. Magnesium stearate was.added in the end and the lubricated compression mixture was pressed into tablets using round punches.

### Example 17: 10 mg orally disintegrating tablets

| | Amount / mg | Amount / % (by weight) |
|---|---|---|
| Aripiprazole hemifumarate | 11.30* | 7.53 |
| Pharmburst^{™} | 132.85 | 88.57 |
| Sucralose | 0.45 | 0.30 |
| Flavour Vanilla | 2.25 | 1.50 |
| Sodium stearyl fumarate | 3.15 | 2.10 |
| Total | 150.00 | 100 |

| | | |
|---|---|---|
| *corresponding to 10 mg of aripiprazole | | |

All ingredients, except magnesium stearate, were homogeneously blended in a suitable mixer. Magnesium stearate was added in the end and the lubricated compression mixture was pressed into tablets using round punches.

### Example 18: 10 mg orally disintegrating tablets

| | Amount / mg | Amount / % (by weight) |
|---|---|---|
| Aripiprazole hemifumarate | 11.30* | 7.53 |
| F-MELT** | 137.20 | 91.47 |
| Sodium stearyl fumarate | 1.50 | 1.00 |
| Total | 150 | 100 |

| | | |
|---|---|---|
| * corresponds to 10 mg of aripiprazole **** F-MELT^{®}C** contains mannitol, xylitol, microcrystalline cellulose, crospovidone and anhydrous dibasic calcium phosphate. F-**MELT^{®}M** contains mannitol, xylitol, microcrystalline cellulose, crospovidone and magnesium aluminometasilicate. Two formulations with a different type of F-MELT were prepared. | | |

Aripiprazole hemifumarate and F-MELT were homogeneously mixed in a suitable mixer. The lubricant sodium stearyl fumarate was added. The obtained compression mixture was compressed into tablets.

### D.) Molding

### Example 19: 10 mg orally disintegrating tablets

| | Amount / mg | Amount / % (by weight) |
|---|---|---|
| Aripiprazole hemifumarate | 11.30* | 7.53 |
| Xylitol | 84.10 | 56.07 |
| Sodium starch glycolate | 4.50 | 3.00 |
| Starch 1500 LM | 48.58 | 32.39 |
| Aspartame | 0.25 | 0.17 |
| Flavour Vanilla | 1.27 | 0.84 |
| Total | 150.00 | 100 |

| | | |
|---|---|---|
| *corresponding to 10 mg of aripiprazole | | |

All ingredients were homogeneously mixed and wetted with purified water and kneaded. The mixture was compression-molded with a round punch to provide tablets. The obtainable tablets were dried.

### IMMEDIATE RELEASE TABLETS:

### A.) Direct compression:

### Tablets of Examples 20 and 21 were prepared by direct compression.

The coloring agent ferric oxide (red) was blended with a small quantity of Cellactose and added to aripiprazole hemifumarate, the remaining part of Cellactose and all other excipients in a suitable mixer. All ingredients were homogeneously blended and compressed into tablets using round punches.

### Example 20: 30 mg immediate release tablets

| | **Amount [mg]** | **Amount by weight [%]** |
|---|---|---|
| Aripiprazole hemifumarate | 33.88* | 7.5 |
| Cellactose | 306.30 | 68.1 |
| Microcrystalline cellulose | 90.00 | 20.0 |
| Croscarmellosse Sodium | 15.00 | 3.3 |
| Magnesium stearate | 4.50 | 1.0 |
| Ferric oxide | 0.32 | 0.07 |
| Total | 450.00 | 100 |

| | | |
|---|---|---|
| *corresponding to 30 mg of aripiprazole | | |

### Example 21: 30 mg immediate release tablets

| | **Amount [mg]** | **Amount by weight [%]** |
|---|---|---|
| Aripiprazole hemifumarate | 33.88* | 7.5 |
| Cellactose | 356.00 | 79.11 |
| Pregelatinized starch | 40.30 | 8.96 |
| Corn starch | 15 | 3.33 |
| Magnesium stearate | 4.50 | 1.00 |
| Ferric oxide | 0.32 | 0.07 |
| Total | 450.00 | 100 |

| | | |
|---|---|---|
| *corresponding to 30 mg of aripiprazole | | |

### B.) Wet granulation:

### Tablets of Example 22 and 23 were prepared by wet granulation.

The mixture of aripiprazole hemifumarate, lactose monohydrate, cornstarch, hydroxypropyl cellulose and ferric oxide was wetted with purified water and kneaded. The granules were dried and sieved. In the end, microcrystalline cellulose and magnesium stearate were added to the dry granulate and the obtained compression mixture was compressed into tablets using round punches.

### Example 22: 30 mg immediate release tablets

| | **Amount [mg]** | **Amount by weight [%]** |
|---|---|---|
| Aripiprazole hemifumarate | 33.88* | 7.5 |
| Lactose monohydrate | 207.06 | 46.0 |
| Corn starch | 51.77 | 11.5 |
| Microcrystalline cellulose | 132.80 | 29.5 |
| Hydroxypropyl cellulose | 19.67 | 4.4 |
| Magnesium stearate | 4.50 | 1.0 |
| Ferric oxide | 0.32 | 0.07 |
| Total | 450.00 | 100 |

| | | |
|---|---|---|
| *corresponding to 30 mg of aripiprazole | | |

### Example 23: 30 mg immediate release tablets

The mixture of aripiprazole hemifumarate and all other ingredients except magnesium stearate was wetted with purified water and kneaded. The wet granules were dried and sieved. In the end magnesium stearate was added to the dry granulate and the obtained compression mixture was compressed into tablets using round punches with 11 mm diameter.

| | **Amount [mg]** | **Amount by weight [%]** |
|---|---|---|
| Aripiprazole hemifumarate | 33.88* | 7.5 |
| Lactose monohydrate | 296.76 | 65.9 |
| Corn starch | 52.06 | 11.6 |
| Microcrystalline cellulose | 52.06 | 11.6 |
| Hydroxypropyl cellulose | 10.41 | 2.3 |
| Magnesium stearate | 4.50 | 1.0 |
| Ferric oxide | 0.32 | 0.07 |
| Total | 450.00 | 100 |

| | | |
|---|---|---|
| *corresponding to 30 mg of aripiprazole | | |

### Example 24: Results of content uniformity of aripiprazole in tablets from example 23

Table 1: Content uniformity of aripiprazole in tablets which are prepared by sieving aripiprazole hemifumarate with a part of diluent before granulation in comparison to the content uniformity in tablets prepared without sieving.

| | **Content of aripiprazole (%)** | |
|---|---|---|
| | **without sieving** | **with sieving** |
| 1 | 104.7 | 100.1 |
| 2 | 103.1 | 100.4 |
| 3 | 107.3 | 103.4 |
| 4 | 100.5 | 104.8 |
| 5 | 103.5 | 100.9 |
| 6 | 107.5 | 101.5 |
| 7 | 101.2 | 101.8 |
| 8 | 104.1 | 101.8 |
| 9 | 102.0 | 101.9 |
| 10 | 103.3 | 103.5 |
| **average** | **103.7** | **102.0** |
| **std %** | **2.31** | **1.5** |
| **RSD** | **2.23** | **1.5** |
| **AV** | **7.74** | **4.1** |

As can be seen, the aripiprazole content in tablets which are prepared by sieving aripiprazole hemifumarate with a part of diluent before granulation is more homogenuously distributed than in tablets without sieving the active ingredient before the granulation process.

### Example 25: Preparation of amorphous aripiprazole hemifumarate

Amorphous form of aripiprazole hemifumarate was prepared by heating crystalline aripiprazole hemifumarate to a temperature of above 203°C followed by cooling in an ice-bath.

## Claims

1. Aripiprazole hemifumarate **characterized in that** the ratio between aripiprazole and fumarate is 2:1.

2. Aripiprazole hemifumarate according to claim 1, **characterized in that** it is crystalline.

3. Aripiprazole hemifumarate according to claim 2, **characterized by** an X-ray powder diffraction pattern having peaks at about 12.1, 15.8, 17.9, 19.7, 21.8 ± 0.2 degrees two-theta.

4. Aripiprazole hemifumarate according to claim 3, which is further **characterized by** having peaks at about 6.7, 8.9, 11.1, 12.1, 15.8, 17.9, 19.7, 21.8, 24.6 ± 0.2 degrees two-theta.

5. Aripiprazole hemifumarate according to claim 2 **characterized by** having a DSC endothermic peak at about 187-192°C.

6. Aripiprazole hemifumarate according to claim 1, **characterized by** an X-ray powder diffraction pattern as in Figure

7. Aripiprazole hemifumarate according to claim 1, **characterized by** a DSC thermogram pattern as in Figure 2.

8. Amorphous aripiprazole hemifumarate according to claim 1.

9. Amorphous aripiprazole hemifumarate, **characterized by** an X-ray powder diffraction pattern as in Figure 6.

10. Process for preparing aripiprazole hemifumarate according to claim 1 comprising:
a) dissolving aripiprazole in a solvent at a temperature of between 15 °C and the reflux temperature of the solvent used;
b) adding fumaric acid in a molar ratio 0.5-0.7 in regard to aripiprazole and heating the mixture to a temperature of between 15 °C and the reflux temperature of the solvent used ;
c) optionally cooling the obtained solution and
d) optionally filtering the precipitated solid.

11. Process according to claim 10, **characterized in that** the order of adding aripiprazole and fumaric acid is reversed and the aripiprazole is added to the fumaric acid.

12. A process according to claim 10 or 11, wherein the solvent is an organic solvent selected from hydrocarbons, halogenated hydrocarbons, ethers, esters, ketones and alcohols; or a combination of these solvents.

13. A process according to claim 12, wherein the organic solvent is ethanol, THF, acetonitrile and/or methylene chloride.

14. Aripiprazole hemifumarate according to claim 1, having an average particle size below 70 micrometers.

15. Aripiprazole hemifumarate according to claim 14, having an average particle size below 50 micrometers.

16. Aripiprazole hemifumarate according to claim 15, having an average particle size below 30 micrometers.

17. Aripiprazole, hemifumarate according to claim 16, having an average particle size below 20 micrometers.

18. Pharmaceutical composition comprising aripiprazole hemifumarate according to any one of claims 1 to 9 or 14 to 17 and pharmaceuticaly acceptable excipients.

19. Pharmaceutical composition according to claim 18, wherein agglomerates of aripiprazole hemifumarate are broken.

20. Pharmaceutical composition according to claim 18, wherein agglomerates of aripiprazole hemifumarate have been sieved.

21. Pharmaceutical composition according to claim 18, which has such a dissolution profile that at least 80% of aripiprazole is dissolved in 0.1M HCl in 30 minutes.

22. Pharmaceutical composition according to claim 18, which has such a dissolution profile that at least 80% of aripiprazole is dissolved in pH 4.5 acetate buffer in 30 minutes.

23. Use of aripiprazole hemifumarate according to any one of claims 1 to 9 or 14 to 17 for the preparation of a pharmaceutical dosage form.

## Patentansprüche

1. Aripiprazolhemifumarat, **dadurch gekennzeichnet, dass** das Verhältnis zwischen Aripiprazol und Fumarat 2:1 ist.

2. Aripiprazolhemifumarat nach Anspruch 1, **dadurch gekennzeichnet, dass** es kristallin ist.

3. Aripiprazolhemifumarat nach Anspruch 2, das durch ein Röntgenpulverbeugungsmuster **gekennzeichnet** ist, das Peaks bei etwa 12,1, 15,8, 17,9, 19,7, 21,8 ± 0,2 Grad 2-Theta aufweist.

4. Aripiprazolhemifumarat nach Anspruch 3, das ferner durch Peaks bei etwa 6,7, 8,9, 11,1, 12,1, 15,8, 17,9, 19,7, 21,8, 24,6 ± 0,2 Grad 2-Theta **gekennzeichnet** ist.

5. Aripiprazolhemifumarat nach Anspruch 2, **dadurch gekennzeichnet, dass** es einen endothermen DSC-Peak bei etwa 187-192°C aufweist.

6. Aripiprazolhemifumarat nach Anspruch 1, das durch ein Röntgenpulverbeugungsmuster wie in Figur 1 **gekennzeichnet** ist.

7. Aripiprazolhemifumarat nach Anspruch 1, das durch ein DSC-Thermogramm wie in Figur 2 **gekennzeichnet** ist.

8. Amorphes Aripiprazolhemifumarat nach Anspruch 1.

9. Amorphes Aripiprazolhemifumarat, das durch ein Röntgenpulverbeugungsmuster wie in Figur 6 **gekennzeichnet** ist.

10. Verfahren zur Herstellung von Aripiprazolhemifumarat nach Anspruch 1, bei dem:
a) Aripiprazol in einem Lösungsmittel bei einer Temperatur zwischen 15°C und der Rückflusstemperatur des verwendeten Lösungsmittels gelöst wird;
b) Fumarsäure in einem Molverhältnis von 0,5 bis 0,7 7 in Bezug auf Aripiprazol zugegeben wird und die Mischung auf eine Temperatur zwischen 15°C und der Rückflusstemperatur des verwendeten Lösungsmittels erhitzt wird;
c) gegebenenfalls die erhaltene Lösung gekühlt wird und
d) gegebenenfalls der gefällte Feststoff filtriert wird.

11. Verfahren nach Anspruch 10, **dadurch gekennzeichnet, dass** die Reihenfolge der Zugabe von Aripiprazol und Fumarsäure umgekehrt ist und das Aripiprazol zu der Fumarsäure gegeben wird.

12. Verfahren nach Anspruch 10 oder 11, bei dem das Lösungsmittel ein organisches Lösungsmittel ausgewählt aus Kohlenwasserstoffen, halogenierten Kohlenwasserstoffen, Ethern, Estern, Ketonen und Alkoholen, oder einer Kombination dieser Lösungsmittel ist.

13. Verfahren nach Anspruch 12, bei dem das organische Lösungsmittel Ethanol, THF, Acetonitiril und/oder Methylenchlorid ist.

14. Aripiprazolhemifumarat nach Anspruch 1, das eine durchschnittliche Partikelgröße unter 70 µm aufweist.

15. Aripiprazolhemifumarat nach Anspruch 14, das eine durchschnittliche Partikelgröße unter 50 µm aufweist.

16. Aripiprazolhemifumarat nach Anspruch 15, das eine durchschnittliche Partikelgröße unter 30 µm aufweist.

17. Aripiprazolhemifumarat nach Anspruch 16, das eine durchschnittliche Partikelgröße unter 20 µm aufweist.

18. Pharmazeutische Zusammensetzung, die Aripiprazolhemifumarat nach einem der Ansprüche 1 bis 9 oder 14 bis 17 und pharmazeutisch annehmbare Hilfsstoffe enthält.

19. Pharmazeutische Zusammensetzung nach Anspruch 18, in der Agglomerate von Aripiprazolhemifumarat zerkleinert sind.

20. Pharmazeutische Zusammensetzung nach Anspruch 18, in der Agglomerate von Aripiprazolhemifumarat gesiebt worden sind.

21. Pharmazeutische Zusammensetzung nach Anspruch 18, deren Auflösungsprofil so beschaffen ist, dass sich mindestens 80% des Aripiprazols in 0,1M HCl in 30 Minuten auflösen.

22. Pharmazeutische Zusammensetzung nach Anspruch 18, deren Auflösungsprofil so beschaffen ist, dass sich mindestens 80% des Aripiprazols in pH 4,5 Acetatpuffer in 30 Minuten auflösen.

23. Verwendung von Aripiprazolhemifumarat nach einem der Ansprüche 1 bis 9 oder 14 bis 17 zur Herstellung einer pharmazeutischen Darreichungsform.

## Revendications

1. Hémifumarate d'aripiprazole, **caractérisé en ce que** le rapport entre aripiprazole et fumarate vaut 2/1.

2. Hémifumarate d'aripiprazole, conforme à la revendication 1, **caractérisé en ce qu'**il est cristallin.

3. Hémifumarate d'aripiprazole, conforme à la revendication 2, **caractérisé en ce que** son diagramme de poudre par diffraction de rayons X comporte des pics situés aux angles 2θ d'environ 12,1°, 15,8°, 17,9°, 19,7° et 21,8°, ± 0,2°.

4. Hémifumarate d'aripiprazole, conforme à la revendication 3, **caractérisé en outre en ce qu'**il donne des pics situés aux angles 2θ d'environ 6,7°, 8,9°, 11,1°, 12,1°, 15,8°, 17,9°, 19,7°, 21,8° et 24,6°, ± 0,2°.

5. Hémifumarate d'aripiprazole, conforme à la revendication 2, **caractérisé en ce qu'**il donne, en analyse thermique différentielle, un pic endothermique situé à environ 187-192 °C.

6. Hémifumarate d'aripiprazole, conforme à la revendication 1, **caractérisé par** un diagramme de poudre par diffraction de rayons X tel que représenté sur la figure 1.

7. Hémifumarate d'aripiprazole, conforme à la revendication 1, **caractérisé par** un thermogramme d'analyse thermique différentielle tel que représenté sur la figure 2.

8. Hémifumarate d'aripiprazole amorphe, conforme à la revendication 1.

9. Hémifumarate d'aripiprazole amorphe, **caractérisé par** un diagramme de poudre par diffraction de rayons X tel que représenté sur la figure 6.

10. Procédé de préparation d'un hémifumarate d'aripiprazole conforme à la revendication 1, comportant les étapes suivantes :
a) dissoudre de l'aripiprazole dans un solvant, à une température située entre 15 °C et la température de reflux du solvant utilisé ;
b) y ajouter de l'acide fumarique, en un rapport molaire à l'aripiprazole de 0,5 à 0,7, et chauffer le mélange à une température située entre 15 °C et la température de reflux du solvant utilisé ;
c) en option, faire refroidir la solution obtenue ;
d) et en option, récupérer par filtration le solide précipité.

11. Procédé conforme à la revendication 10, **caractérisé en ce qu'**on inverse l'ordre d'addition de l'aripiprazole et de l'acide fumarique, et qu'on ajoute donc l'aripiprazole à l'acide fumarique.

12. Procédé conforme à la revendication 10 ou 11, dans lequel le solvant est un solvant organique choisi parmi les hydrocarbures, hydrocarbures halogénés, éthers, esters, cétones et alcools, ou une combinaison de tels solvants.

13. Procédé conforme à la revendication 12, dans lequel le solvant organique est de l'éthanol, du tétrahydrofurane, de l'acétonitrile et/ou du chlorure de méthylène.

14. Hémifumarate d'aripiprazole, conforme à la revendication 1, en particules dont la taille moyenne est inférieure à 70 micromètres.

15. Hémifumarate d'aripiprazole, conforme à la revendication 14, en particules dont la taille moyenne est inférieure à 50 micromètres.

16. Hémifumarate d'aripiprazole, conforme à la revendication 15, en particules dont la taille moyenne est inférieure à 30 micromètres.

17. Hémifumarate d'aripiprazole, conforme à la revendication 16, en particules dont la taille moyenne est inférieure à 20 micromètres.

18. Composition pharmaceutique comprenant un hémifumarate d'aripiprazole, conforme à l'une des revendications 1 à 9 et 14 à 17, et des excipients pharmacologiquement admissibles.

19. Composition pharmaceutique conforme à la revendication 18, pour laquelle les agglomérats d'hémifumarate d'aripiprazole ont été désagrégés.

20. Composition pharmaceutique conforme à la revendication 18, pour laquelle les agglomérats d'hémifumarate d'aripiprazole ont été éliminés par tamisage.

21. Composition pharmaceutique conforme à la revendication 18, qui présente un profil de dissolution tel qu'au moins 80 % de l'aripiprazole se dissout en 30 minutes dans de l'acide chlorhydrique à 0,1 M.

22. Composition pharmaceutique conforme à la revendication 18, qui présente un profil de dissolution tel qu'au moins 80 % de l'aripiprazole se dissout en 30 minutes dans une solution tampon d'acétate à pH 4,5.

23. Utilisation d'un hémifumarate d'aripiprazole à l'une des revendications 1 à 9 et 14 à 17 en vue de la préparation d'une forme pharmaceutique.
